# EUROPEAN PATENT APPLICATION

(11) **EP 1 818 413 A1**
(43) Date of publication of application: **15.08.2007**
(21) Application number: 06075283.9
(22) Date of filing: 10.02.2006
(51) Int. Cl.: C12Q 1/68

(54) **Method of determining outcome of non small-cell lung cancer according to XRCC3 polymorphism**

(71) Applicant: Fundacion para la Investigacion Clinica y Molecular del Cancer de Pulmon, 08036 Barcelona (ES)
(72) Inventor: Rosell Costa, Rafael, 08916 Badalona (ES); Tarón Roca, Miguel, 08916 Badalona (ES)
(74) Representative: ABG Patentes, S.L.

(57) **Abstract**

Method for determining a platinum-based chemotherapeutic regimen for treating Non-Small-Cell Lung cancer (NSCLC) in a patient comprising:
a) determining the presence or absence of the XRCC3 MetMet polymorphism in a biological sample from the patient,
c) determining a platinum-based chemotherapeutic regimen based on the presence or absence of the XRCC3 MetMet polymorphism in the sample,
wherein the presence of said polymorphism is indicative of favourable predisposition of said subject to respond to a chemotherapy treatment comprising the combination gemcitabine/cisplatin.

SEQUENCE LISTING

<110> FUNDACIÓN CÁNCER DE PULMÓN

<120> METHOD OF DETERMINING OUTCOME OF NON SMALL-CELL LUNG CANCER
ACCORDING TO XRCC3 POLYMORPHISM

<140> EP06075283.9
<141> 2006-02-10

<160> 4

<210> 1
<211> 17
<212> DNA
<213> Artificial sequence

<220> Synthetic DNA
<223> Forward primer designed for SNP genotyping using allelic
Discrimination Assay

<400> 1
ccagggccag gcatctg 17

<210> 2
<211> 18
<212> DNA
<213> Artificial sequence

<220> Synthetic DNA
<223> Reverse primer designed for SNP genotyping using allelic
Discrimination Assay

<400> 2
cagcacaggg ctctggaa 18

<210> 3
<211> 15
<212> DNA
<213> Artificial sequence

<220> Synthetic DNA
<223> Probe designed for SNP genotyping using allelic Discrimination
Assay

<400> 3
cagcatggcc cccca 15

<210> 4
<211> 14
<212> DNA
<213> Artificial sequence

<220> Synthetic DNA
<223> Probe designed for SNP genotyping using allelic Discrimination
Assay

<400> 4
cagcgtggcc ccca                                                          14

## Description

### FIELD OF THE INVENTION

The present invention relates to the field of diagnostics, in particular to methods for obtaining a prognosis for a subject suffering from Non-Small Cell Lung Cancer, as well as for identifying those subjects having a greater benefit from treatment with platinum based chemotherapy by the determination of a XRCC3 polymorphism, and to the assessment and/or treatment of such patients.

### BACKGROUND OF THE INVENTION

Lung cancer is the leading cause of cancer-related mortality in both men and women. Lung cancer recently surpassed heart disease as the leading cause of smoking-related mortality. Non-small cell lung cancer (NSCLC) accounts for approximately 75% of all lung cancers. NSCLC is a heterogeneous aggregate of histologies the most common ones being epidermoid or squamous carcinoma, adenocarcinoma, and large cell carcinoma.

Despite international efforts spent in the investigation of novel "targeted agents" for the treatment of lung cancer, platinum-based cytotoxic doublets such as cisplatin plus gemcitabine remain the standard first-line treatment for patients with advanced disease. Cisplatin or cis diamminedichloroplatinum (DDP) is an inorganic compound that is widely used for the treatment of a variety of tumors (germ-cell, advanced bladder carcinoma, adrenal cortex carcinoma, breast cancer, head and neck carcinoma, lung carcinoma). Gemcitabine is a chemotherapy drug that is given as a treatment for some types of cancer. It is most commonly used to treat NSCLC, pancreatic, bladder and breast cancer.

Currently, cisplatin (DDP) and carboplatin are among the most widely used cytotoxic anticancer drugs. However, resistance to these drugs through de novo or induced mechanisms undermines their curative potential. These drugs disrupt DNA structure through formation of intrastrand adducts. Resistance to platinum agents such as DDP has been attributed to enhanced tolerance to platinum adducts, decreased drug accumulation, or enhanced DNA repair.

Response rates with cisplatin are variable, with higher rates in adenocarcinoma, female gender, oriental origin and never-smoker status. Unfortunately, survival rate varies significantly between individual patients, with some patients surviving years, and others succumbing to their disease within a few months.

Several studies have attempted to identify clinical, laboratory, and molecular markers that may help clinicians and researchers distinguish subgroups of NSCLC patients. Nevertheless, relatively few prognostic factors, such as extent of disease and performance status, have been widely accepted as useful prognostic markers. Furthermore, there is no simple and reliable way to estimate the survival of individual patients undergoing chemotherapy.

Therefore, it is necessary to identify clinical factors that influence the outcome of advanced NSCLC patients treated with standard chemotherapy, and to build a model that can be used in daily practice to predict long-term survival in this patient population. Such a practical model may help oncologists and their patients make treatment decisions, chose the most appropriate chemotherapy, and may assist investigators in designing clinical trials.

### SUMMARY OF THE INVENTION

Surprisingly, discoveries by the inventors have shed light on the relationship between genotype and sensitivity to cisplatin-gemeitabine and cisplatin-docetaxel chemotherapy. We have found that XRCC3 241 MetMet is strongly associated with survival in cisplatin-gemcitabine treated non-small-cel-lung cancer patients. Further, we have found that this association is very significant in patients under 55 years of age, and is reduced as the age increases. This finding implies that the corresponding polymorphism assay will be useful for the adequate selection of chemotherapy.

Accordingly, the present invention provides a novel method to determine the likelihood of effectiveness of a platinum based chemotherapeutic regime, such as cisplatin-gemeitabine and cisplatin-docetaxel combination chemotherapy, in a human patient affected with NSCLC.

In a first aspect the invention is directed to a method for determining a platinum-based chemotherapeutic regimen for treating Non-Small-Cell Lung Cancer (NSGLC) in a patient comprising:
a) determining the presence or absence of the XRCC3 MetMet polymorphism in a biological sample from the patient,
c) determining a a platinum-based chemotherapeutic regimen based on the presence or absence of the XRCC3 MetMet polymorphism in the sample.

In a second aspect the invention provides a method for evaluating the predisposition of a patient suffering from NSCLC to respond to a chemotherapy treatment comprising the combination gemcitabine/cisplatin, which comprises determining the presence or absence of the polymorphism 241MetMet of XRCC3 in a sample from said subject, wherein the presence of said polymorphism is indicative of favourable predisposition of said subject to respond to said chemotherapy treatment.

In a third aspect, the invention is directed to a method for determining a platinum-based chemotherapeutic regimen for treating Non-Small-Cell Lung cancer (NSCLC) in a patient comprising:
a) obtaining a sample from the patient,
b) determining the presence or absence of the XRCC3 MetMet polymorphism in the sample from the patient,
c) determining a a platinum-based chemotherapeutic regimen based on the presence or absence of the XRCC3 MetMet polymorphism in the sample,
wherein the presence of said polymorphism is indicative of favourable predisposition of said subject to respond to a chemotherapy treatment comprising the combination gemeitabine/cisplatin.

In a fourth aspect, the invention provides a method for designing an individual chemotherapy treatment based on cisplatin-gemcitabine for a subject suffering from NSCLC which comprises:
i) determine the existence of the polymorphism 241 MetMet of XRCC3 in a sample from said subject;
ii) considering the data obtained in the previous step for designing an individual chemotherapy treatment, so that the presence of said polymorphism is indicative of predisposition of thc subject to be treated with a chemotherapy treatment comprising the combination gemcitabine/cisplatin.

In a fifth aspect, the invention comprises the use of a combination comprising gemcitabine/cisplatin in the preparation of a medicament for the treatment of a patient suffering from NSCLC and presenting the polymorphism 241MetMet of XRCC3.

In a sixth aspect, the invention provides the use of an effective amount of a chemotherapy comprising a combination of gemcitabine/cisplatin for the treatment of a patient suffering from NSCLC and presenting the polymorphism 241MetMet of XRCC3.

### BRIEF DESCRIPTION OF THE FIGURES

**Figure 1** shows the Kaplan Meicr curves for survival according to XRCC3 241 genotype in a study involving 135 patients.
**Figure 2** shows the Kaplan Meier curves for survival of patients bearing the XRCC3 241 MetMet genotype, broken down according to age, from a study involving 651 patients.

### DETAILED DESCRIPTION OF THE INVENTION

The term"polymorphism"as used herein refers to the occurrence of two or more genetically determined alternative sequences or alleles in a population. A single nucleotide polymorphism occurs at a polymorphic site occupied by a single nucleotide, which is the site of variation between allelic sequences. A single nucleotide polymorphism usually arises due to substitution of one nucleotide for another at the polymorphic site.

The term "genotype" in the context of this invention refers to the particular allelic form of a gene, which can be defined by the particular nucleotide (s) present in a nucleic acid sequence at a particular site (s).

In the context of this invention, the term"probe"refers to a molecule which can detectably distinguish between target molecules differing in structure. Detection can be accomplished in a variety of different ways depending on the type of probe used and the type of target molecule. Thus, for example, detection may be based on discrimination of activity levels of the target molecule, but preferably is based on detection of specific binding. Examples of such specific binding include antibody binding and nucleic acid probe hybridization. Thus, for example, probes can include enzyme substrates, antibodies and antibody fragments, and preferably nucleic acid hybridization probes.

The term"primer", as used herein, refers to an oligonucleotide which is capable of acting as a point of initiation of polynucleotide synthesis along a complementary strand when placed under conditions in which synthesis of a primer extension product which is complementary to a polynucleotide is catalyzed. Such conditions include the presence of four different nucleotide triphosphates or nucleoside analogs and one or more agents for polymerization such as DNA polymerase and/or reverse transcriptase, in an appropriate buffer ("buffer"includes substituents which are cofactors, or which affect pI-I, ionic strength, etc.), and at a suitable temperature. A primer must be sufficiently long to prime the synthesis of extension products in the presence of an agent for polymerase. A typical primer contains at least about 5 nucleotides in length of a sequence substantially complementary to the target sequence, but somewhat longer primers arc preferred.

Gemcitabine is the generic name assigned to 2'-deoxy-2',2'-difluoro-cytidine. It is commercially available as the monohydrochloride salt, and as the beta-isomer. It is also known chemically as 1-(4-amino-2-oxo-1H-pyrimidin-1 -yl)-2-dcsoxy-2,2-difluororibose. Gemcitabine is disclosed in U.S. Pat. Nos. 4,808,614 and 5,464,826, which are incorporated herein by reference. Gemcitabine is administered at doses comparable to those routinely utilized clinically- For example, the initial dose of gemcitabine, typically as the hydrochloride salt, will be about 1000-1250 mg/m² of body surface area. This product is routinely formulated as a sterile solution and is administered by intravenous infusion, generally over about a 30-minute period, with about 2 to 4 weekly doses, with courses repeated about every 28 to 30 days. The dose of 1000-1250 mg/m² can be given for up to about 7 weeks, according to this treatment regimen, or until undesirable side effects arc observed. Other salt forms can be utilized if desired, for example, the hydrobromide, monophosphate, sulfate, malonate, citrate, and succinate are readily prepared.

Cisplatin is the generic name for cis-diaminodichloroplatinum and is described in U.S. Pat. No. 5,562,925, which is incorporated herein by reference. Cisplatin generally is formulated as a sterile solution for injection, and is routinely administered at a dose of about 50 to 100 mg/m², given intravenously. This cycle can be repeated for about every 4 to 8 weeks.

The DNA within our cells is continually being exposed to DNA-damaging agents. These include ultraviolet light, natural and man-made mutagenic chemicals and reactive oxygen species generated by ionizing radiation or by processes such as redox cycling by heavy metal ions and radio-mimetic drugs. Of the various forms of damage that are inflicted by these mutagens, probably the most dangerous is the DNA double-stand break (DSB). DNA DSBs arc generated when the two complementary stands of the DNA double helix arc broken simultaneously at sites that are sufficiently close to one another that base-pairing and chromatin structure are insufficient to keep to two DNA ends juxtaposed. As a consequence, the two DNA ends generated by a DSB arc liable to become physically dissociated from one another, making ensuring repair difficult to perform and providing the opportunity for inappropriate recombination with other sites in the genome. Despite posing major threats to genomic integrity, DSBs arc nevertheless sometimes generated deliberately and for a defined biological purpose. DSBs are potent inducers of cell death. One cellular response to DSBs is to activate and/or induce the levels of DNA repair proteins, which arc then physically recruited to the site of the DNA lesion to bring about its repair.

Double-strand breaks (DSBs) are repaired by homologous recombination (HR) and non-homologous endjoining (NHEJ). HR can occur by gene conversion with or without an associated crossover. Crossovers can result in chromosomal rearrangements including deletions, inversions, translocations, and large-scale loss of heterozygosity. HR is generally a high-fidelity repair mechanism, because crossovers are largely suppressed in mitotic cells. IIR proteins include RAD51, the RAD51 paralogs (XRCC2, XHCC3), BRCA1, and BRCA2.

The X-ray repair cross-complementing group 3 (XRCC3) functions in the repair of DNA double-strand breaks by homologous recombination. In mammalian cells, mutations of XRCC3 reduce DSB-induced HR by 30- to >250 fold. Furthermore, XRCC3 has been proposed to play a role in stabilizing hetcroduplcx DNA (hDNA) which are produced during the reparation mechanism.

The polymorphism in codon 241 (Thr to Met) of XRCC3, defined in the present description as polymorphism 241 MetMet of XRCC3, has been associated with the level of bulky DNA adducts in leukocytes of healthy subjects. Carriers of XRCC3 241 MetMet had higher levels of DNA adducts regardless of smoking status, leading us to hypothesize that an inefficient DNA repair mechanism in these patients could make them more chemosensitive. In a recent study, individuals with XRCC3 241 MetMet showed a higher risk of developing lung cancer.

Cisplatin is still the scaffolding of combination chemotherapy in non-small cell lung cancer (NSCLC). Results tend to be similar whether the partner drug is paclitaxel, docetaxel, or gemcitabine. Similar results are generally obtained with carboplatin, although in a randomized study, median survival was 8.2 months in the paclitaxcl/carboplatin arm and 9.8 months in the paclitaxel/cisplatin arm.

Platinum-based chemotherapies cause a"bulky adduct"of the DNA, wherein the primary effect is to distort the three-dimensional conformation of the double helix. Such compounds are meant to be administered alone, or together with other chemotherapies such as gemcitabine (Gem) or 5-Fluorouracil (5-FU). They comprise heavy metal coordination compounds which form covalent DNA adducts. Generally, these heavy metal compounds bind covalently to DNA to form, in pertinent part, cis-1, 2-intrastrand dinucleotide adducts. Generally, this class is represented by cis-diamminedichloroplatinum (II) (cisplatin).

Polymorphic variants in DNA repair genes can explain inter-individual differences in survival in cisplatin-treated non-small-cell lung cancer patients independently of performance status, the primary clinical prognostic factor. Single nucleotide polymorphisms can impair the DNA repair mechanisms involved in removing DNA adducts through DNA double-strand break/recombination repair, nucleotide excision repair, base excision repair and non-homologous end joining. Understanding the correlation between DNA repair genotypes and survival in non-small-cell lung cancer can help to elucidate how certain polymorphic variants can adversely or favorably influence chemotherapy outcome.

It has been described that there is a reduced DNA repair capacity resulting from genetic polymorphisms of various DNA repair genes and that this circumstance is associated with improved survival in subjects treated with platinum based chemotherapy. However, there is no relation established between the DNA repair capacity and the response to different platinum based combination therapies. To clarify the impact of the 241 MetMet polymorphism in the XRCC3 gene, we examined the relationship between the different genotypes and survival in stage IV non-small-cell lung cancer patients receiving gemcitabine plus cisplatin versus those receiving docetaxel plus cisplatin. Surprisingly, we found that there is a clear correlation.

In one aspect the invention is directed to a method for determining a platinum-based chemotherapeutic regimen for treating Non-Small-Cell Lung Cancer (NSCLC) in a patient comprising:
a) determining the presence or absence of the XRCC3 MetMet polymorphism in a biological sample from the patient,
c) determining a a platinum-based chemotherapeutic regimen based on the presence or absence of the XRCC3 MetMet polymorphism in the sample.

The method of the invention in its different embodiments will be described now in detail.

First a sample tissue or body fluid of a patient suffering from NSCLC is taken. The present method can be applied to any type of tissue or body fluid from a patient.

In one embodiment it is preferable to examine tumor tissue. Preferably this is done prior to the chemotherapy. Tumors or portions thereof are surgically resected from the patient or obtained by routine biopsy. To simplify conservation and handling of the samples, these can be formalin-fixed and paraffin-embedded.

However, from the clinical point of view, the obtention of tissue samples is limited because of the scarcity of tumor tissue obtained by bronchoscopy in stage IV NSCLC patients. In early stages, sometimes we can benefit from the resected tumor specimens that provide tumor tissue for RNA extraction. But a much better alternative is to use body fluids, in particular serum, as the sample.

Genetic analysis has shown that cell-free circulating DNA in plasma or serum of cancer patients shares similar genetic alterations to those described in the corresponding tumor.

Therefore, in a preferred embodiment of the the sample is a body fluid from the NSCLC patient selected from blood, plasma or scrum. More preferably it is serum. Serum is easily and immediately available from the patient, it suffices to take a blood sample and separate the cells by centrifugation.

In accordance with the invention, the process of "determining the presence or absence of the XRCC3 MetMet polymorphism" in a biological sample from a patient may advantageously comprise screening for the presence or absence in the genome of the subject of both the common allele and the variant allele or may comprise screening for the presence or absence of the MetMet allele, it generally being possible to draw conclusions about the genotype of an individual at a polymorphic locus just by screening for one of the specific alleles.

The step of determining the genotype of a NSCLC patient at the 241 XRCC3 polymorphic locus, also referred to as "genotyping", can be carried out using any suitable methodology known in the art and it is to be understood that the invention is in no way limited by the precise technique used to perform such genotyping.

The nucleic acids, preferably DNA, are extracted from the sample by procedures known to the skilled person and commercially available such as the QIAmp Blood Mini kit of QIAGEN.

Known techniques for the scoring of single nucleotide polymorphisms (see review by Schafer, A. J. and Hawkins: "DNA variation and the future of human genetics", Nature Biotechnology, 1998 Jan(16) 33-39) include among others mass spectrometry, particularly matrix-assisted laser desorption/ionization time-of-flight mass spectrometry (MALDI-TOF-MS), single nucleotide primer extension and DNA chips or microarrays. The use of DNA chips or microarrays could enable simultaneous genotyping the simultaneous genotyping of the single polymorphic locus in multiple individuals.

In addition to the above, SNPs are commonly scored using PCR-based techniques, such as PCR-SSP using allele-specific primers. This method generally involves performing DNA amplification reactions using genomic DNA as the template and two different primer pairs, the first primer pair comprising an alicic-specific primer which under appropriate conditions is capable of hybridising selectively to the wild type allele and a non allele-specific primer which binds to a complementary sequence elsewhere within the gene in question, the second primer pair comprisingan-allele- specific primer which under appropriate conditions is capable of hybridising selectively to the variant allele and the same non allele-specific primer. A still further technique for scoring SNPs is the so-called PCR ELISA technique. SNPs may also be scored by DNA sequencing.

Preferably, the 5' nuclease allelic discrimination assay is used. Further details about the determination of the polymorphism using this method can be found in the examples, where the primers and probes used arc given, good results were obtained with such sequences. Other primers and probes readily apparent to the person skilled in the art can be used.

Once the presence or absence of the XRCC3 MetMet polymorphism is established, the determination of the platinum based chemotherapy can be done following the findings of the inventors that XRCC3 241 MetMet is both an easily assessable and robust predictive marker for survival in younger gemcitabine/cisplatin treated NSCLC patients.

Therefore, if the polymorphism is present, gemcitabine/cisplatin will be the treatment of choice. This is clearer for patients under 57 year of age, and even more under 55 years of age. Thse patients are more likely to show a clinical response to the gemcitabine/cisplatin treatment and show longer survival.

As used herein,"a clinical response" is the response of the tumor to treatment with a chemotherapeutic agent. Criteria for determining a response to therapy are widely accepted and enable comparisons of the efficacy alternative treatments. A complete response (or complete remission) is the disappearance of all detectable malignant disease. A partial response is an approximately 50 percent decrease in the product of the greatest perpendicular diameters of one or more lesions, no new lesions and no progression of any lession. A responder is a patient giving a complete or partial response to the cisplatin or carboplatin chemotherapy.

The invention being thus described, practice of the invention is illustrated by the experimental examples provided below. These examples should not be interpreted as limiting the scope of the claims.

### EXAMPLES

### Example 1

### XRCC3 241 MetMet is associated with longer survival in cisplatin-gemcitabine-treated NSCL cancer patients

The inventors assessed polymorphisms in the peripheral blood of stage IV non-small-cell lung cancer patients and correlated genotypes with survival. The study was approved by the independent ethics committees of all participating centers, and all patients gave their signed informed consent.

### Methodology

### Subjects

Patients were included in a Spanish Lung Cancer Group multicenter clinical trial. Patients were considered eligible if they had stage IV or stage IIIB (with malignant pleural effusion) histologically confirmed non-small-cell lung cancer. Other eligibility criteria included an Eastern Cooperative Oncology Group performance status of 0 (asymptomatic and fully active) or 1 (symptomatic, fully ambulatory, restricted in physically strenuous activity); age of at least 18 years; adequate hematologie function (hemoglobin at least 9 g per deciliter [5.6 mmol per liter], neutrophil count at least 1500 per cubic millimeter, and platelet count at least 100,000 per cubic millimeter); adequate renal function (serum creatinine less than 1-5 times the upper limit of normal); and adequate liver function (bilirubin not more than 1.5 times the upper limit of normal, aspartate aminotransferase and alanine aminotransferase not more than 5 times the upper limit of normal). Patients with clinically overt brain metastases and those who had received previous chemotherapy were excluded. Patients with a performance status of 2 (symptomatic, ambulatory, capable of self-care, more than 50 percent of walking hours spent out of bed) were also excluded, based on results of previous studies where these patients had a high rate of serious adverse events and poor survival.

### Treatment

Patients received cisplatin at a dose of 75 mg per square meter of body-surface area on day 1 plus gemcitabine at a dose of 1250 mg per square meter on days 1 and 8. The cycle was repeated every 3 weeks for a maximum of six cycles.

Survival was calculated from the date of enrollment to the date of death or last clinical follow-up.

### Sample collection and genotyping

Venous blood (10 ml) was collected from each subject into tubes containing 50 mmol of EDTA (ethylenediaminetetraacetic acid) per liter, and genomic DNA was isolated with the QIAmp® DNA blood Mini kit (Qiagen, Germany), according to manufacturer's instructions. Polymorphisms were assessed using the 5' nuclease allelic discrimination assay, In this method, the region Banking the polymorphism is amplified by PCR in the presence of two probes, each specific for one or the other allele. The primers and probes used for SNP gcnotyping using allelic Discrimination Assay were:
Forward primer (5'→3'): CCAGGGCCAGGCATCTG
Reverse primer (5'→3'); CAGCACAGGGCTCTGGAA
Nucleotide T→Met [M] Probe ((5'→3'): CAGC**A**TGGCCCCCCA
Nucleotide C→Thr [T] Probe (5'→3'): CAGC**G**TGGCCCCCA

Each oligonucleotide probe is 5' labeled with a different fluorescent reporter dye (FAM or VIC) to differentiate the amplification of each allele, and with a quencher dye. During PCR, each probe anneals specifically to complementary sequences between the forward and reverse primer sites. DNA polymerase can cleave only probes that fully hybridize to the allele. Cleavage separates the reporter dye from the quencher dye, which results in increased fluorescence by the reporter dye. The PCR-generated fluorescent signal(s) indicate(s) the alleles that are present in the sample. Each reaction mixture (12.5 µL) of polymerase chain reaction contained 50 ng of DNA, 900 nM of each forward and reverse primer, 300 nM of each allele specific probe, and 6.25 µL of TaqMan Universal PCR Master Mix (Applied Biosystems, Foster City, CA). Amplification was done under the following conditions: 50° C for 2 minutes, 95° C for 10 minutes followed by 40 cycles of 92° C for 15 seconds and 60° C for 1 minute. Fluorescence in each sample well was measured before and after PCR using ABI Prism 7900HT Sequence Detection System (Applied Biosystems). Data were analyzed using Allelic Discrimination Program (Applied Biosystems). For each polymorphism, a minimum of 20 randomly selected DNA samples were genotyped at least twice to confirm the results.

### Statistical Analyses

The endpoint of the study was overall survival, calculated from the start of treatment to the date of last follow-up or death. Demographic and clinical variables were compared across genotype, using Fisher's exact test or the Pearson chi square test for categorical variables and the one-way analysis of variance for continuous and normally distributed variables or the Kruskall-Wallis test for non-normal variables. Normality was checked by the Kolmogorov-Smirnov test.

To verify the agreement of the observed genotype frequencies with those expected according to the Hardy-Weinberg equilibrium model, the likelihood-ratio test G was used. Both univariate and multivariate logistic regression models were used to calculate either crude or adjusted odds ratios of response to treatment. Survival curves were plotted using the Kaplan and Meier method and compared with the log-rank test. Median follow-up time was computed for all patients alive at the time of analysis. Multivariate Cox proportional hazard models adjusting genotypes for performance status, smoking status and age were used to perform forward and backward stepwise regression analyses to identify prognostic factors for survival. The association between genotypes and survival was estimated by computing the hazard ratios and their 95 percent confidence intervals from both univariate and multivariate Cox regression models, where the most frequent allele was assumed to be the reference. Statistical significance was set at 5%. All tests were two-sided and analyses were carried out with SPSS software, version 11.5 (Chicago, IL).

### Results

A total of 135 patients were enrolled in this study. The median follow-up for all patients was 9.7 months (range, 0.4-30.7). Patient characteristics are shown in the following table

**Table 1**

| | **N(%)** |
|---|---|
| **Age, years (median, range)** | 62 (31-81) |
| **Number of cycles (median, range)** | 6 (1-7) |
| **Sex** | |
| **Male** | 125 (92.6) |
| **Female** | 10 (7.4) |
| **Smoking status** | |
| **Smokers (current or ex-smokers)** | 113 (83.7) |
| **Never smokers** | 22(16.3) |
| **Performance status** | |
| **0** | 38(28.1) |
| **1** | 97 (71.9) |
| **Histological type** | |
| **Adenocarcinoma** | 63 (47) |
| **Squamous cell carcinoma** | 46 (34.3) |
| **Large cell carcinoma** | 25(18.7) |

Median age was 62 years (range, 31-81); 92 percent were male; 28 percent had performance status 0; 83 percent were smokers; and 47 percent had adenocarcinoma. No patient had received thoracic radiotherapy. The distribution of all genotypes (wild-type, heterozygous and homozygous polymorphic variants) and allelic frequencies are shown in table 2:

| **Polymorphism** | **Genotypic frequencies (%)** | | | **Allelic frequencies** | |
|---|---|---|---|---|---|
| XRCC3 T241M | ThrThr | ThrMet | MetMet | Thr | Met |
| | 39.2 | 42.3 | 18.4 | 0.60 | 0.40 |

Genotype frequencies were consistent with previously reported studies and were in agreement with those expected according to the Hardy-Weinberg equilibrium model. There was no significant correlation between genotype and age, performance status, smoking status, gender or histology.

### Survival

Median survival for all 135 patients was 10.90 months (95 percent confidence interval, 8.82 to 13). Median survival was 15.54 months for patients with performance status 0 and 9.51 for those with performance status 1 (hazard ratio, 0.46; 95 percent confidence interval 0.28 to 0.77; P-0.003). Median survival was 10.63 months for smokers while it was not reached for non-smokers (hazard ratio, 0.51; 95 percent confidence interval 0.26 to 1.02, P=0.06). No other correlation between survival and clinical or demographic characteristics was observed.

### Polymorphisms and survival

Median survival was 15.56 months for carriers of XRCC3 241 MetMet, 13.95 (9.89-18.01) months for those with ThrThr and 9.61(7.33-11.88) months for those with ThrMet (log-rank test, P-0.01) The univariate Cox regression model showed that XRCC3 241 MetMet significantly correlated with prolonged survival (hazard ratio: XRCC3 241 MetMet, 0.43; 95 percent confidence interval, 0.22 to 0.82; P=0.01 when compared to XRCC3 241 ThrMet). The stepwise multivariate Cox regression model including performance status, smoking status, and XRCC3 241 identified only XRCC3 241 and performance status as independent prognostic factors for survival.

### Survival adjusted for performance status

In the Cox proportional hazards model adjusted for performance status, significant differences in survival were observed for the variant genotypes of XRCC3 241.

Compared to carriers of XRCC3 241 ThrMet, the adjusted hazard ratio was significantly lower for patients with MetMet (hazard ratio, 0.44; 95 percent confidence interval, 0.23 to 0.84; P=0.01) and nearly significantly lower for those with ThrThr (hazard ratio, 0.64; 95 percent confidence interval, 0.40 to 1.02; P=0.06). In the multivariate model including performance status and smoking status, the hazard ratios for XRCC3 241 MetMet and ThrThr remained the same as in the univariate model, indicating that XRCC3 is an independent prognostic factor for survival.

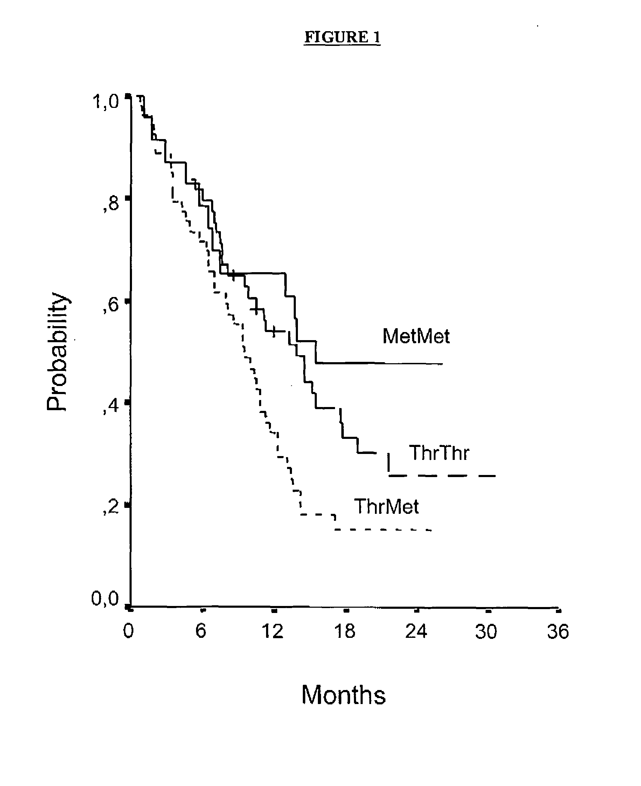

Our results show that XRCC3 is strongly associated with survival in cisplatin-gemcitabine-treated non-small-cell lung cancer patients. Figure 1 shows the Kaplan Meier curves for survival according to XRCC3 241 genotype.

The fact that XRCC3 241 MetMet predicts prolonged survival with cisplatin-gemcitabine makes useful the development of polymorphism assays for selection of chemotherapy.

### Example 2

Following the procedures described in example 1, a total of 651 stage IV NSCLC patients receiving docetaxel (Taxotere) plus cisplatin (474 patients) or gemcitabine plus cisplatin (177) were evaluated. The characteristics of the patients are summarised in table 3:

| | **Doc/Cis N (%)** | **Gem/Cis N (%)** | **p** |
|---|---|---|---|
| **No Patients** | **474** | **177** | |
| Age | | | **0.01** |
| **Median** | **59.7** | **62** | |
| **Range** | **30.6-79.5** | **31-82** | |
| **Sex** | | | **0.001** |
| **Male** | **396 ( 83.5)** | **165 (93.2)** | |
| **Female** | **78 (16.5)** | **12(6.8)** | |
| **PS** | | | **0.84** |
| **0** | **131 (27.6)** | **47 (26.6)** | |
| **1** | **343 (72.4)** | **130 (73.4)** | |
| **Histology** | | | **0.06** |
| **Adeno** | **239 (50.9)** | **75 (42.6)** | |
| **SCC** | **147 (31.3)** | **73 (41.5)** | |
| **LCC** | **84 (17.9)** | **28 (15.9)** | |
| **Stage** | | | **0.35** |
| **IIIB** | **73 (15.4)** | **22 (12.4)** | |
| **IV** | **401 (84.6)** | **155 (87.6)** | |
| **Surgery (yes)** | **42(8.9)** | **16 (9)** | **0.99** |
| **Radiotherapy (yes)** | **38 (8)** | **14 (7.9)** | **0.99** |

The distribution of the genotypes found in stage IV NSCLC patients are shown in table 4, distributed according to the chemotherapy given to them:

| **Polymorphism** | **Genotypic frequencies p (%)** | | |
|---|---|---|---|
| XRCC3 T241M | ThrThr | ThrMet | MetMet |
| Docetaxcl/cis | 125 (35.8) | 173(49.6) | 51 (14.6) |
| Gemcitabine/cis | 65 (38) | 75(43.9) | 31 (18.1) |

Overall median survival (MS) was 9.6 months. For patients treated with gemcitabine/cisplatin Median Survival was 16.7 for XRCC3 241 MetMet, 12.3 for ThrThr and 10.4 for ThrMet. For patients treated with docetaxel/cisplatin Median Survival was 8.4 for XRCC3 241 MetMet, 9.4 for ThrThr and 9.5 for ThrMet.

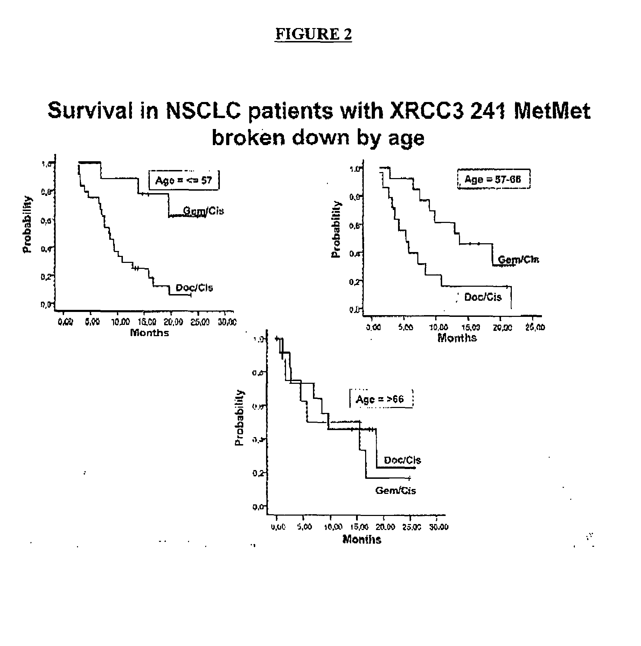

Significantly, in patients with XRCC3 241 MetMet and under 57 years of age, Median Survival was not reached for patients treated with gemcitabine/cisplatin and was 8.4 months for patients treated with docetaxcl/cisplatin (P=0.02). This difference diminished in patients with XRCC3 241 MetMet in the range of 57-66 years (MS 13.7 months with gemcitabine/cisplatin, 5.4 months with docetaxel/cisplatin); and nearly disappeared in patients above 66 years (MS 5.8 months with gemcitabine/cisplatin, 9.6 months with docctaxcl/cisplatin). Figure 2 shows the Kaplan Meier curves for survival of patients bearing the XRCC3 241 Met Met genotype, broken down according to age. The difference in survival according to age is clear. Therefore XRCC3 241 MetMet genotype is an excellent predictive marker for gemcitabine/cisplatin except in elderly patients.

### Example 3

Following the procedure explained in example 1, real-time PCR assay was used to determine XRCC3 genotype from DNA isolated from baseline blood samples of 878 stage IV Non Small Cell Lung Cancer patients (162 treated with gemcitabine/cisplatin; 716 with docetaxel/cisplatin). The patient characteristics arc as follows: median age, 60; 266 patients (30%) <55; 239 patients (39%) 55-66; 273 patients (31%) >66. Adenocarcinoma: 459 patients (53%).

Homozygous variant XRGC3 241 MetMet was found in 124 patients (14%), with the same frequency in each of the three age groups.

After a median follow-up of 7.6 months (95%Cl, 1-47 months), overall median survival (MS) was 9.5 months (95%Cl, 8.8-10.2 m), with no differences between the 2 regimens.

In all patients with XRCC3 241 MetMet, Median Survival was 12.9 months for patients treated with gemcitabine/cisplatin and 8.4 months for patients treated with docetaxel/cisplatin (P=0,06) (hazard ratio at 2 y - 0.23). In patients with XRCC3 241 MetMet and under 55 years of age, Median Survival was not reached For patients treated with gemcitabine/cisplatin and was 9.2 months for patients treated with docctaxcl/cisplatin (P=0.02), which translated into a 60% difference in survival at 2 years.

This difference diminished in patients with XRCC3 241 MetMet in the range of 55-66 years (MS 12.9 months with gemcitabine/cisplatin, 6.9 months with docetaxel/cisplatin [P=0.09]; 28% difference in survival at 2 years) and disappeared in patients above 66 years of age (MS 5.8 months with gemcitabine/cisplatin, 7.8 months with docetaxel/cisplatin [P=0.55].

For the other XRCC3 241 genotypes (ThrThr and ThrMet), no differences in MS were found either overall or broken down by age.

Conclusions: XRCC3 241 MetMet is both an easily assessable and robust predictive marker for survival in younger gemcitabine/cisplatin treated NSCLC patients. The survival benefit dwindles with increasing age, possibly related to the enhanced DNA repair capacity of older patients.

## Claims

1. Method for determining a platinum-based chemotherapeutic regimen for treating Non-Small-Cell Lung cancer (NSCLC) in a patient comprising:
a) determining the presence or absence of the XRCC3 MetMet polymorphism in a biological sample from the patient,
b) determining a a platinum-based chemotherapeutic regimen based on the presence or absence of the XRCC3 MetMet polymorphism in the sample.

2. The method according to claim 1 wherein the sample is form a patient under 57 years of age.

3. The method according to claim 1 or 2 wherein the sample is a blood sample.

4. The method according to claim 1 wherein the platinum-based chemotherapeutic regimen is either a combination of gemcitabine/cisplatin or a combination of docetaxel/cisplatin.

5. The method according to claim 4, wherein when the polymorphism XRCC3 MetMet is present the platinum-based chemotherapeutic regimen is determined to be a combination of gemcitabine/cisplatin.

6. Method for evaluating the predisposition of a patient suffering from NSCLC to respond to a chemotherapy treatment comprising the combination gemcitabine/cisplatin which comprises determine the presence or absence of the polymorphism 241 MetMet of XRCC3 in a sample from said subject, wherein the presence of said polymorphism is indicative of favourable predisposition of said subject to respond to said chemotherapy treatment.

7. The method according to claim 6 wherein the patient is under 57 years of age.

8. Method for determining a platinum-based chemotherapeutic regimen for treating Non-Small-Cell Lung cancer (NSCLC) in a patient comprising:
a) obtaining a sample from the patient,
b) determining the presence or absence of the XRCC3 MetMet polymorphism in the sample from the patient,
c) determining a a platinum-based chemotherapeutic regimen based on the presence or absence of the XRCC3 MetMet polymorphism in the sample,
wherein the presence of said polymorphism is indicative of favourable predisposition of said subject to respond to a chemotherapy treatment comprising the combination gemcitabine/cisplatin.

9. The method according to claim 8, wherein the determining step comprises analyzing a nucleic acid present in the sample.

10. The method according to claim 1, wherein the determining step comprises analyzing a protein present in the sample.

11. The method according to any of claims 1-10, wherein the sample is a blood sample.

12. Method for designing an individual chemotherapy treatment based on cisplatin-gemeitabine for a subject suffering from NSCLC which comprises:
i) determine the existence of the polymorphism 241 MctMct of XRCC3 in a sample from said subject;
ii) considering the data obtained in the previous step for designing an individual chemotherapy treatment, so that the presence of said polymorphism is indicative of predisposition of the subject to be treated with a chemotherapy treatment comprising the combination gemcitabine/cisplatin.

13. Use of a combination comprising gemcitabine/cisplatin in the preparation of a medicament for the treatment of a patient suffering from NSCLC and presenting the polymorphism 241 MetMet of XRCC3.

14. Use according to claim 13 wherein the NSCLC patient is under 57 years of age.

15. Use of an effective amount of a chemotherapy comprising a combination of gemcitabine/cisplatin for the treatment of a patient suffering from NSCLC and presenting the polymorphism 241 MetMet of XRCC3.
